# EUROPEAN PATENT APPLICATION

(11) **EP 0 953 365 A2**
(43) Date of publication of application: **03.11.1999**
(21) Application number: 98121487.7
(22) Date of filing: 12.11.1998
(51) Int. Cl.: A61M 39/10

(54) **Female connector**

(30) Priority: 01.05.1998 JP 13780398
(71) Applicant: Kawasumi Laboratories, Inc., Tokyo 140 (JP)
(72) Inventor: Ono, Seiichi c/o Kawasumi Laboratories, Inc., Ono-gun, Oita 879-7153 (JP); Watanabe, Hiroaki c/o Kawasumi Laboratories, Inc., Minamiamabe-gun, Oita 876-0121 (JP); Otuka, Tetuya c/o Kawasumi Laboratories, Inc., Ono-gun, Oita 879-7153 (JP)
(74) Representative: Grünecker, Kinkeldey, Stockmair & Schwanhäusser Anwaltssozietät

(57) **Abstract**

A female connector 1A includes a core member 1, and a housing 2 mounted on an outer periphery of the core member 1, and the core member 1 has a luer taper 4 formed on an inner surface thereof, and has projections 5 and 5A formed on an outer surface thereof. The housing 2 has a thread ridge 7 formed on an outer surface thereof, and has recesses 8 and 8A which are formed on an inner surface thereof, and correspond respectively to the projections 5 and 5A. The core member 1 is inserted into a hole, defined by the inner surface of the housing 2, without being fixedly bonded to the inner surface of the housing.

## Description

### BACKGROUND OF THE INVENTION

### 1. Field of the Invention

This invention relates to a female connector provided at an end of various branch tubes for a blood circuit, an administration tube, a blood transfusion tube, various catheter tubes and other medical fluid guide tubes, and more particularly to such a female connector which can be enhanced in operability and performance when it is used.

### 2. Description of the Related Art

A female connector, provided at an end of a fluid guide tube, can be connected to a male connector provided at other tube or an instrument. A predetermined luer taper is formed on an inner surface of the female connector, and a predetermined thread is formed on an outer surface thereof. The male connector has a double concentric cylindrical-wall construction, and a predetermined luer taper is formed on an outer surface of an inner cylinder thereof, and a predetermined thread is formed on an inner surface of an outer cylinder thereof. The threads of the two connectors, as well as the luer tapers thereof, are fitted together, thereby achieving liquid-tightness and air-tightness.

The female connector is classified into a one-part type, in which the female connector is molded as one piece, and a two-part type in which a luer taper portion and a threaded portion are respectively formed in two parts, and are combined together into a unitary construction by bonding, pressing, fitting, insert molding or other method. In both types, the female connector, when to be connected to the male connector, is inserted thereinto while being rotated, and therefore a tube, connected to the female connector, is twisted, so that it can not be used easily. Besides, the luer taper portions of the two connectors are connected together in a twisted condition, and therefore can not be completely brought into intimate contact with each other, which has often resulted in liquid leakage and air leakage. In the case where the female connector, molded into a one-part construction, is used in a blood circuit or the like, a colorant is used for distinguishing between an artery side and a vein side, and therefore there is a possibility that the colorant dissolves out into a fluid from a liquid-contact portion. Besides, when the tube was forcibly folded or bent, liquid leakage and air leakage, in some cases, have been occurred.

### SUMMARY OF THE INVENTION

It is therefore an object to the present invention to provide a female connector that can be positively fixed to a male connector with air-tightness and liquid-tightness, and prevents a tube from twisting when it is used.

According to the present invention, a female connector includes a core member, and a housing mounted on an outer periphery of the core member, and the core member has a luer taper formed on an inner surface thereof, and has projections formed on an outer surface thereof. The housing has a thread ridge formed on an outer surface thereof, and has recesses which are formed on an inner surface thereof, and correspond respectively to the projections. The core member is inserted into a hole, defined by the inner surface of the housing, without being fixedly bonded to the inner surface of the housing.

### BRIEF DESCRIPTION OF THE DRAWINGS

Similar reference characters denote corresponding features consistently throughout the attached drawings. The preferred embodiments of this invention will be described in detail, with reference to the following figures, wherein:
Fig. 1 is a cross-sectional view of a female connector of the present invention;
Fig. 2 is a cross-sectional view taken along the line A-A' of Fig. 1;
Fig. 3 is a cross-sectional view showing an example of use in which the female connector of Fig. 1 is connected to a male connector;
Fig. 4 is a cross-sectional view showing another embodiment of a female connector of the invention;
Fig. 5 is a cross-sectional view showing a further embodiment of a female connector of the invention;
Fig. 6 is a cross-sectional view showing a still further embodiment of a female connector of the invention.

### DETAILED DESCRIPTION OF THE PREFERRED EMBODIMENTS

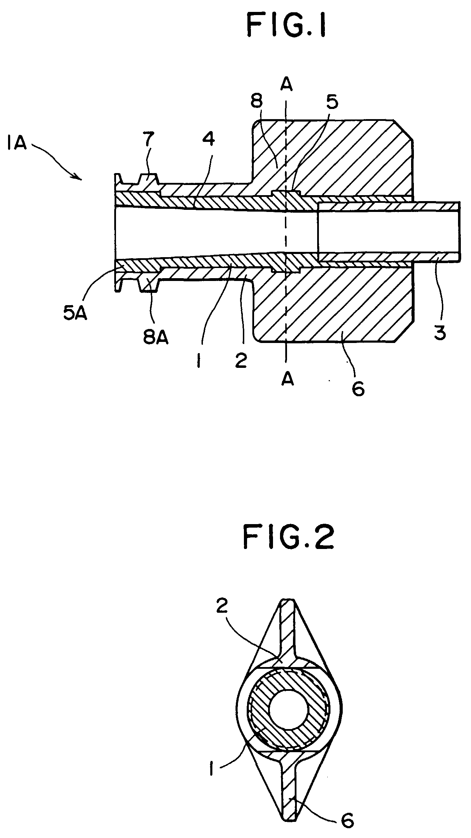

Fig. 1 is a cross-sectional view of a female connector 1A of the present invention, and the female connector 1A includes a core member 1, and a housing 2 mounted on an outer periphery of the core member 1. A fluid guide tube 3 is connected to a rear end portion of the core member 1.

The core member 1 and the fluid guide tube 3 are made of a soft thermoplastic material or a semi-rigid thermoplastic material such for example as polyvinyl chloride and polyurethane, and the housing 2 is made of a rigid thermoplastic material such for example as polypropylene and polycarbonate.

The core member 1 is formed into a cylindrical shape, and the fluid guide tube 3 is connected to an inner surface of the rear end portion thereof, and a luer taper 4, for example, on the order of 6/100, is formed on an inner surface of that portion of the core member extending from a central portion thereof to a front end thereof. Projections 5 and 5A are formed respectively at the central portion and front end portion of the core member at the outer surface thereof.

The housing 2 is also formed into a cylindrical shape, and a thread ridge 7 is formed on an outer surface thereof at a front end portion thereof, and recesses 8 and 8A, corresponding respectively to the projections 5 and 5A, are formed in an inner surface thereof respectively at a central portion thereof and the front end portion thereof.

Wings 6 may be formed on the outer surface of the housing 2 over an area, extending from the central portion thereof to the rear end thereof, so that the housing can be easily held by the fingers when in operation.

The core member 1 and the housing 2 are respectively produced by injection molding, and the fluid guide tube 3 is produced by extrusion.

For example, the outer surface of the front end portion of the fluid guide tube 3 is connected by bonding to the inner surface of the rear end portion of the core member 1 of the female connector 1A, and then that portion of the core member 1, extending from its central portion to its front end, is softened by heating, and then is inserted into the housing 2 in concentric relation thereto without being fixedly bonded to the inner surface of the housing 2 by an adhesive, thermal welding or the like, and by doing so, the female connector 1A can be easily assembled.

Alternatively, the core member 1 may be first inserted into the housing 2 as described above, and finally the fluid guide tube 3 may be connected by bonding to the housing 2.

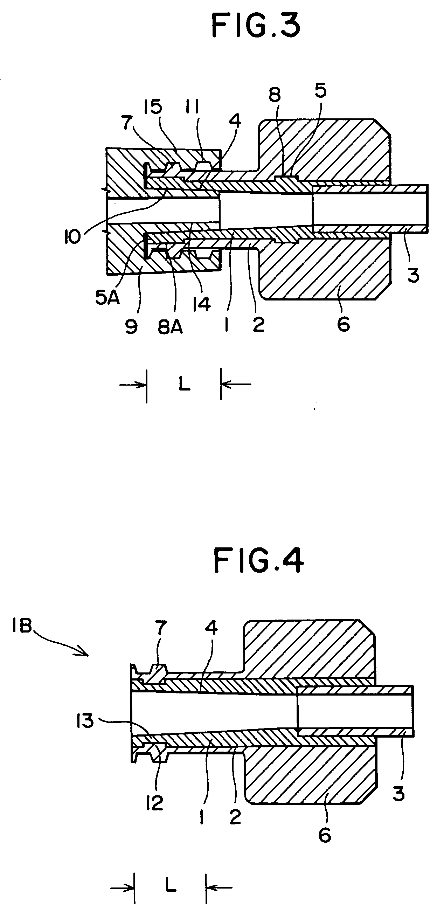

Next, one example of a method of using the female connector 1A of the present invention will be described with reference to Fig. 3.

In the drawings, reference numeral 9 denotes a male connector, and like the housing 2, this male connector 9 is made of a rigid material, and is formed into a double concentric cylindrical-wall construction having an inner cylinder 14 and an outer cylinder 15. A luer taper 10 is formed on an outer surface of the inner cylinder 14, and a thread groove 11 is formed in an inner surface of the outer cylinder 15.

When the female connector 1A is to be connected to the male connector 9, the thread ridge 7 on the housing 2 is positioned relative to the thread groove 11 in the male connector 9, and the housing is rotated, so that the thread ridge 7 is tightened along the thread groove 11. At this time, the projection 5A on the core member 1 are forced toward a rear end of the male connector 9 through the recess 8A in the housing 2, and therefore the luer taper 4 of the core member 1 is held in contact with the luer taper 10 of the male connector 9, and in this condition the core member 1 is slidingly inserted in the direction of the axis of the inner cylinder 14 without being rotated.

The core member 1 is made of a soft material or a semi-rigid material, and therefore the luer taper 4 is pressed against the luer taper 10 of the male connector 9, so that the female connector 1A and the male connector 9 are connected together in intimate contact with each other.

For disconnecting the female connector 1A from the male connector 9, the housing 2 is rotated in a direction opposite to the direction for the connecting operation. By thus rotating the housing in the opposite direction, the thread ridge 7 is loosened along the thread groove 11, and the projection 5A on the core member 1 is brought out of the male connector 9 through the recess 8A in the housing 2.

If the core member 1 of the female connector 1A of Fig. 1 is made of an extremely soft material, the projection 5 is deformed in the axial direction to be disengaged from the recess 8 when disconnecting the female connector 1A from the male connector 9, which leads to a possibility that only the housing 2 is removed, with the core member 1 kept connected to the male connector 9. To avoid this, it can provide a female connector 1B (which is another embodiment of a female connector of the present invention) having a configuration shown in Fig. 4.

In the female connector 1B, the provision of the recesses 8 and 8A in the housing 2 of the female connector 1A is omitted, and instead a projection 12 is formed on an inner surface of a front end portion of a housing 2, and also the provision of the projections 5 and 5A on the core member 1 of the female connector 1A is omitted, and instead a recess 13 is formed in an outer surface of a front end portion of a core member 1. The projection 12 and the recess 13 are formed respectively at such positions that they are disposed in a region L of contact between a luer taper 4 of the core member 1 and the luer taper 10 of the male connector 9 when the female connector 1B is connected to the male connector 9. With the above position of the projection 12 and the recess 13, the housing 2 is rotatable in a radial direction relative to the core member 1 and is not disengaged in an axial direction from the core member 1.

The positions of formation of the projection 12 and the recess 13 are not limited to those shown in Fig. 4 in so far as the core member 1 and the housing 2 are rotatable relative to each other in the radial direction on each cylindrical surface and the housing 2 is not be disengaged from the core member 1, and the projection 12 and the recess 13 can be formed at any position in so far as they can be disposed in the region L of contact between the luer taper 4 of the core member 1 and the luer taper 10 of the male connector 9. A method of using the female connector 1B is substantially the same as that described above for the female connector 1A, and therefore detailed explanation thereof will be omitted here.

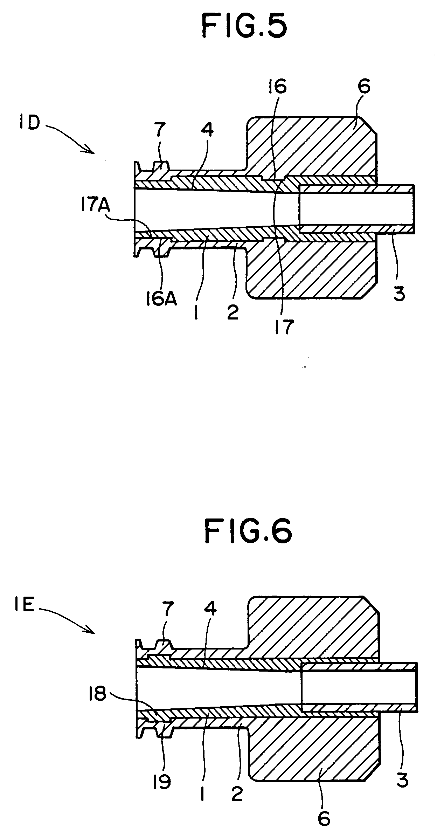

Further, the projections 5 and 5A, formed on the core member 1 of the female connector 1A of Fig. 1, and the recesses 8 and 8A, formed in the housing 2, may be replaced with each other as in a female connector 1D of Fig. 5, in which case recesses 17 and 17A are formed in a core member 1 while projections 16 and 16A are formed on a housing 2. Similarly, the recess 13, formed in the core member 1 of the female connector 1B of Fig. 4, and the projection 12, formed on the housing 2, may be replaced with each other as in a female connector 1E of Fig. 6, in which case a projection 18 is formed on a core member 1 while a recess 19 is formed in a housing 2. A method of using the female connectors 1D and 1E is substantially the same as that described above for the female connector 1A, and therefore detailed description thereof will be omitted here.

### [Advantageous Effects of the Invention]

The female connector 1A (1B, 1D, 1E) is assembled by inserting the core member 1, having the luer taper 4, into the housing 2, having the thread ridge 7, in a concentric manner without bonding the core member 1 to the housing 2, and therefore the core member 1 and the housing 2 are rotatable relative to each other in the radial direction on each cylindrical surface, and is not disengaged from each other in the axial direction.

For connecting the female connector to the male connector 9, the thread ridge 7 of the housing 2, while rotated, is received in the thread groove 11, and the luer taper 4 of the core member 1 slides in the axial direction, and is brought into intimate contact with the luer taper 10 of the male connector 9. Therefore the connection portions of the female connector 1A (1B, 1D, 1E) and the male connector 9 are positively fixed together.

Therefore, (a) in use, the fluid guide tube 3 will not be twisted. (b) The luer taper 4 is not be twisted at the time of the insertion, and is held straight in intimate contact, and therefore liquid leakage and air leakage are prevented. (c) Because of the two-part construction, even when a load is applied to the fluid guide tube 3, the luer taper 4 is always held in intimate contact. (d) For distinguishing between an artery side and a vein side in the case where the female connector of a two-part construction is used in a blood circuit or the like, the liquid-contact portion of the two-part connector is not colored whereas only the non-liquid-contact portion can be colored, and therefore a colorant or the like will not dissolve out into a fluid in contrast with the conventional one-part product.

The entire disclosure of each and every foreign patent application from which the benefit of foreign priority has been claimed in the present application is incorporated herein by reference, as if fully set forth.

While only certain embodiments of the invention have been specifically described herein, it will apparent that numerous modifications may be made thereto without departing from the spirit and scope of the invention.

## Claims

1. A method of assembling a female connector (1A, 1B, 1D, 1E) comprising the steps of:
providing a core member (1) and a housing (2), said core member (1) having a luer taper surface (4) on an inner surface thereof and formed with at least one engaging portion (5, 5A; 13; 17, 17A; 18) on an outer surface thereof, said housing (2) having a thread ridge (7) on an outer surface thereof and formed with at least one engaged portion (8, 8A; 12; 16, 16A; 19) corresponding to said engaging portion (5, 5A; 13; 17, 17A; 18) on an inner surface thereof;
heating a front portion of said core member (1), so as to soften said core member (1);
inserting said core member (1) into said housing (2) so that said engaging portion (5, 5A; 13; 17, 17A; 18) coincides with said engaged portion (8, 8A; 12; 16, 16A; 19), to thereby prevent the relative movement between said core member (1) and said housing (2) in an axial direction of said core member (1).

2. A method according to claim 1, wherein said engaging portion is a projection (5, 5A; 18), and said engaged portion is a recess (8, 8A; 16A) engaging with said projection (5, 5A; 18).

3. A method according to claim 1, wherein said engaging portion is a recess (13; 17, 17A), and said engaged portion is a projection (12; 16, 16A) engaging with said recess (13; 17, 17A).

4. A female connector (1A, 1B, 1D, 1E) comprising:
a core member (1) having a luer taper surface (4) on an inner surface thereof; and
a housing (2) mounted on an outer surface of said core member (1) and formed with a thread ridge (7) on an outer surface thereof,
characterized in that:
at least one projection (5, 5A; 12; 16, 16A; 18) is formed on one of the outer surface of said core member (1) and an inner surface of said housing (2),
at least one recess (8, 8A; 13; 17, 17A; 19) engagable with said projection (5, 5A; 12; 16, 16A; 18) is formed on the other of the outer surface of said core member (1) and the inner surface of said housing (2), and
said core member (1) is inserted into the inner surface of said housing (2) without fixedly bonding with said housing (2).

5. A female connector (1A, 1B, 1D, 1E) according to claim 4, wherein said projection (5, 5a; 18) is formed on the outer surface of said core member (1), and said recess (8, 8A; 19) is formed on the inner surface of said housing (2).

6. A female connector (1A, 1B, 1D, 1E) according to claim 4, wherein said recess (13; 17, 17A) is formed on the outer surface of said core member (1), and said projection (12; 16, 16A) is formed on the inner surface of said housing (2).

7. A female connector (1A, 1B, 1D, 1E) according to claim 4, wherein said core member (1) is made of a soft or a rigid thermoplastic member.

8. A connector unit comprising:
a first connector (9) formed in a double concentric cylinder shape so as to have an inner cylinder (14) and an outer cylinder (15), said inner cylinder (14) having a luer taper surface (10) on an outer surface thereof, said outer cylinder (15) being formed with a thread groove (11) on an outer surface thereof,
a second connector (1A, 1B, 1D, 1E) fitting with said first connector, said second connector comprising:
a core member (1) having a luer taper surface (4) on an inner surface thereof; and
a housing (2) mounted on an outer surface of said core member (1) and formed with a thread ridge (7) fitting with said thread groove (11) on an outer surface thereof,
characterized in that:
at least one projection (5, 5A; 12; 16, 16A; 18) is formed on one of the outer surface of said core member (1) and an inner surface of said housing (2),
at least one recess (8, 8A; 13; 17, 17A; 19) engagable with said projection (5, 5A; 12; 16, 16A; 18) is formed on the other of the outer surface of said core member (1) and the inner surface of said housing (2), and
said core member (1) is inserted into the inner surface of said housing (2) without fixedly bonding with said housing (2).

9. A connector unit according to claim 8, wherein said projection (5A; 12; 16A; 18) and said recess (8A; 13; 17A; 19) are located in a contact region L in which said luer taper surface (10) of said first connector (9) contacts with said luer taper surface (4) of said second connector (1A, 1B, 1D, 1E).
